(19)

Europäisches
Patentamt
European
Patent Office
Office européen
des brevets

(11) **EP 2 399 604 A1**

(12) **EUROPEAN PATENT APPLICATION**

(43) Date of publication:
28.12.2011 Bulletin 2011/52

(51) Int Cl.:
*A61K 39/395* (2006.01)   *C07K 16/28* (2006.01)
*A61P 11/06* (2006.01)

(21) Application number: 10167357.2

(22) Date of filing: 25.06.2010

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **F. Hoffmann-La Roche AG
4070 Basel (CH)**

(72) Inventor: **The designation of the inventor has not
yet been filed**

(74) Representative: **Heiroth, Ulrike Hildegard
F. Hoffmann-La Roche AG
Patent Department, Grenzacherstrasse 124
CH-4070 Basel (CH)**

(54) **Novel antibody formulation**

(57)   The present invention relates to a pharmaceutical liquid formulation of an antibody against human OX40L, a
process for the preparation and uses of the formulation.

EP 2 399 604 A1

SEQUENCE LISTING

<110>  F. Hoffmann-La Roche AG

<120>  Novel Antibody Formulation

<130>  NDR9725

<160>  3

<170>  PatentIn version 3.5

<210>  1
<211>  214
<212>  PRT
<213>  Artificial Sequence

<220>
<223>  light chain of OX40L antibody

<400>  1

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Tyr Pro Tyr
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
            115                 120                 125

Thr Ala Ser Val Val Cys Leu Leu Asn Asn Phe Tyr Pro Arg Glu Ala
        130                 135                 140

Lys Val Gln Trp Lys Val Asp Asn Ala Leu Gln Ser Gly Asn Ser Gln
145                 150                 155                 160
```

```
Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
                180                 185                 190

Ala Cys Glu Val Thr His Gln Gly Leu Ser Ser Pro Val Thr Lys Ser
                195                 200                 205

Phe Asn Arg Gly Glu Cys
        210


<210>  2
<211>  450
<212>  PRT
<213>  Artificial Sequence

<220>
<223>  heavy chain of Mab1

<400>  2

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Asn Ser Tyr
                20                  25                  30

Ala Met Ser Trp Val Arg Gln Ala Pro Gly Lys Gly Leu Glu Trp Val
            35                  40                  45

Ser Ile Ile Ser Gly Ser Gly Gly Phe Thr Tyr Tyr Ala Asp Ser Val
        50                  55                  60

Lys Gly Arg Phe Thr Ile Ser Arg Asp Asn Ser Arg Thr Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Leu Val Ala Pro Gly Thr Phe Asp Tyr Trp Gly Gln
            100                 105                 110

Gly Ala Leu Val Thr Val Ser Ser Ala Ser Thr Lys Gly Pro Ser Val
            115                 120                 125

Phe Pro Leu Ala Pro Ser Ser Lys Ser Thr Ser Gly Gly Thr Ala Ala
        130                 135                 140
```

```
Leu Gly Cys Leu Val Lys Asp Tyr Phe Pro Glu Pro Val Thr Val Ser
145             150             155             160

Trp Asn Ser Gly Ala Leu Thr Ser Gly Val His Thr Phe Pro Ala Val
            165             170             175

Leu Gln Ser Ser Gly Leu Tyr Ser Leu Ser Ser Val Val Thr Val Pro
            180             185             190

Ser Ser Ser Leu Gly Thr Gln Thr Tyr Ile Cys Asn Val Asn His Lys
            195             200             205

Pro Ser Asn Thr Lys Val Asp Lys Lys Val Glu Pro Lys Ser Cys Asp
    210             215             220

Lys Thr His Thr Cys Pro Pro Cys Pro Ala Pro Glu Leu Leu Gly Gly
225             230             235             240

Pro Ser Val Phe Leu Phe Pro Pro Lys Pro Lys Asp Thr Leu Met Ile
            245             250             255

Ser Arg Thr Pro Glu Val Thr Cys Val Val Val Asp Val Ser His Glu
            260             265             270

Asp Pro Glu Val Lys Phe Asn Trp Tyr Val Asp Gly Val Glu Val His
            275             280             285

Asn Ala Lys Thr Lys Pro Arg Glu Glu Gln Tyr Asn Ser Thr Tyr Arg
    290             295             300

Val Val Ser Val Leu Thr Val Leu His Gln Asp Trp Leu Asn Gly Lys
305             310             315             320

Glu Tyr Lys Cys Lys Val Ser Asn Lys Ala Leu Pro Ala Pro Ile Glu
            325             330             335

Lys Thr Ile Ser Lys Ala Lys Gly Gln Pro Arg Glu Pro Gln Val Tyr
            340             345             350

Thr Leu Pro Pro Ser Arg Asp Glu Leu Thr Lys Asn Gln Val Ser Leu
            355             360             365

Thr Cys Leu Val Lys Gly Phe Tyr Pro Ser Asp Ile Ala Val Glu Trp
    370             375             380

Glu Ser Asn Gly Gln Pro Glu Asn Asn Tyr Lys Thr Thr Pro Pro Val
385             390             395             400
```

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
                420                 425                 430

Glu Ala Leu His Asn His Tyr Thr Gln Lys Ser Leu Ser Leu Ser Pro
                435                 440                 445

Gly Lys
    450


<210>  3
<211>  450
<212>  PRT
<213>  Artificial Sequence

<220>
<223>  heavy chain of Mab2

<400>  3

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Asn Ser Tyr
                20                  25                  30

Ala Met Ser Trp Val Arg Gln Ala Pro Gly Lys Gly Leu Glu Trp Val
                35                  40                  45

Ser Ile Ile Ser Gly Ser Gly Gly Phe Thr Tyr Tyr Ala Asp Ser Val
                50                  55                  60

Lys Gly Arg Phe Thr Ile Ser Arg Asp Asn Ser Arg Thr Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Leu Val Ala Pro Gly Thr Phe Asp Tyr Trp Gly Gln
                100                 105                 110

Gly Ala Leu Val Thr Val Ser Ser Ala Ser Thr Lys Gly Pro Ser Val
                115                 120                 125

Phe Pro Leu Ala Pro Ser Ser Lys Ser Thr Ser Gly Gly Thr Ala Ala
                130                 135                 140

```
Leu Gly Cys Leu Val Lys Asp Tyr Phe Pro Glu Pro Val Thr Val Ser
145             150             155             160

Trp Asn Ser Gly Ala Leu Thr Ser Gly Val His Thr Phe Pro Ala Val
                165             170             175

Leu Gln Ser Ser Gly Leu Tyr Ser Leu Ser Ser Val Val Thr Val Pro
            180             185             190

Ser Ser Ser Leu Gly Thr Gln Thr Tyr Ile Cys Asn Val Asn His Lys
            195             200             205

Pro Ser Asn Thr Lys Val Asp Lys Lys Val Glu Pro Lys Ser Cys Asp
    210             215             220

Lys Thr His Thr Cys Pro Pro Cys Pro Ala Pro Glu Leu Leu Gly Gly
225             230             235             240

Pro Ser Val Phe Leu Phe Pro Pro Lys Pro Lys Asp Thr Leu Met Ile
            245             250             255

Ser Arg Thr Pro Glu Val Thr Cys Val Val Val Asp Val Ser His Glu
            260             265             270

Asp Pro Glu Val Lys Phe Asn Trp Tyr Val Asp Gly Val Glu Val His
    275             280             285

Asn Ala Lys Thr Lys Pro Arg Glu Glu Gln Tyr Asn Ser Thr Tyr Arg
    290             295             300

Val Val Ser Val Leu Thr Val Leu His Gln Asp Trp Leu Asn Gly Lys
305             310             315             320

Glu Tyr Lys Cys Lys Val Ser Asn Lys Ala Leu Pro Ala Pro Ile Glu
            325             330             335

Lys Thr Ile Ser Lys Ala Lys Gly Gln Pro Arg Glu Pro Gln Val Tyr
            340             345             350

Thr Leu Pro Pro Ser Arg Glu Glu Met Thr Lys Asn Gln Val Ser Leu
        355             360             365

Thr Cys Leu Val Lys Gly Phe Tyr Pro Ser Asp Ile Ala Val Glu Trp
    370             375             380

Glu Ser Asn Gly Gln Pro Glu Asn Asn Tyr Lys Thr Thr Pro Pro Val
385             390             395             400
```

```
Leu Asp Ser Asp Gly Ser Phe Phe Leu Tyr Ser Lys Leu Thr Val Asp
                405                 410                 415


Lys Ser Arg Trp Gln Gln Gly Asn Val Phe Ser Cys Ser Val Met His
            420                 425                 430


Glu Ala Leu His Asn His Tyr Thr Gln Lys Ser Leu Ser Leu Ser Pro
            435                 440                 445


Gly Lys
    450
```

**Description**

The present invention relates to a pharmaceutical liquid formulation of an antibody against human OX40L, a process for the preparation and uses of the formulation.

Human antibodies against human OX40 ligand (OX40L, gp34, Swiss Prot P23510) are described in WO2006/029879. These antibodies inhibit the interaction of OX40L with OX40 in an ELISA using immobilized OX40L at a coating concentration of 0.5 μg/ml with an IC50 value of no more than 4 nM.

Low concentration formulations, in liquid form, lyophilized form or in liquid form reconstituted from a lyophilized form, of antibodies against OX40L are disclosed in WO2009/141239.

The invention relates to a pharmaceutical liquid formulation comprising:

　　　1 to 200 mg/mL of an antibody;
　　　1 to 100 mM of a buffer;
　　　0.001 to 1% of a surfactant;

　　　　　(a) 5 to 500 mM of a stabilizer; or
　　　　　(b) 5 to 500 mM of a stabilizer and 5 to 500 mM of a tonicity agent; or
　　　　　(c) 5 to 500 mM of a tonicity agent;

　　　at a pH in the range of from 4.0 to 7.0,
　　　wherein the antibody is an antibody against OX40 ligand.

The invention relates to a pharmaceutical liquid formulation of a human antibody binding to human OX40L comprising

1 to 200 mg/ml of said antibody comprising as light chain the light chain of SEQ ID NO:1 and as heavy chain the heavy chain of SEQ ID NO:2 or 3;
1 to 100 mM of a buffer;
0.001 to 1% of a surfactant;

　　　　　(a) 5 to 500 mM of a stabilizer; or
　　　　　(b) 5 to 500 mM of a stabilizer and 5 to 500 mM of a tonicity agent; or
　　　　　(c) 5 to 500 mM of a tonicity agent;

at a pH in the range of from 4.0 to 7.0.

The term "liquid" as used herein in connection with the formulation according to the invention denotes a formulation which is liquid at a temperature of at least about 2 to about 8°C under atmospheric pressure.

The term "human antibody against human OX40L" as used herein denotes an antibody comprising as light chain the light chain of SEQ ID NO:1 and as heavy chain the heavy chain of SEQ ID NO:2 (further named Mab1) or as light chain the light chain of SEQ ID NO:1 and as heavy chain the heavy chain of SEQ ID NO:3 (further named Mab2). The antibody is an IgG1 kappa type antibody. The antibody binds to human OX40 ligand (OX40L, gp34, Swiss Prot P23510). The antibody according to the invention binds to OX40L with a KD value of $10^{-12}$ to $10^{-9}$ M in a BIA core assay.

The term "buffer" as used herein denotes a pharmaceutically acceptable excipient, which stabilizes the pH of a pharmaceutical preparation. Suitable buffers are well known in the art and can be found in the literature. Examples of pharmaceutically acceptable buffers are histidine-buffers, citrate-buffers, acetate-buffers, arginine-buffers or mixtures thereof, e.g. L-histidine or mixtures of L-histidine and L-histidine hydrochloride with pH adjustment with an acid or a base known in the art. Independently from the buffer used, the pH may be adjusted at a value in the range of from about 4.0 to about 7.0, e.g. from about 5.0 to about 6.5 or from about 5.5 to about 6.5, e.g. pH 5.3 or pH $5.8_2$ with an acid or a base known in the art, e.g. hydrochloric acid, acetic acid, and citric acid and sodium hydroxide.

The term "surfactant" as used herein denotes a pharmaceutically acceptable excipient which is used to protect protein formulations against mechanical stresses like agitation and shearing. Examples of pharmaceutically acceptable surfactants include polyoxyethylensorbitan fatty acid esters (Tween) and polyoxyethylene-polyoxypropylene copolymer (Poloxamer, Pluronic). Examples of polyoxyethylenesorbitan-fatty acid esters are polysorbate 20 (sold under the trademark Tween 20™) and polysorbate 80 (sold under the trademark Tween 80™). Examples of polyethylene-polypropylene copolymers are those sold under the names Pluronic® F68 or Poloxamer 188™. Examples of polyoxyethylene alkyl ethers are those sold under the trademark Brij™.

The term "stabilizer" denotes a pharmaceutical acceptable excipient, which protects the active pharmaceutical ingredient and/or the formulation from chemical and/or physical degradation during manufacturing, storage and application. Chemical and physical degradation pathways of protein pharmaceuticals are reviewed by Cleland et al. (1993), Crit Rev Ther Drug Carrier Syst 10(4):307-77, Wang (1999) Int J Pharm 185(2):129-88, Wang (2000) Int J Pharm 203 (1-2):1-60 and Chi et al. (2003) Pharm Res 20(9):1325-36. Stabilizers include but are not limited to sugars, amino acids, polyols, cyclodextrines, e.g. hydroxypropyl-β-cyclodextrine, sulfobutylethyl-β-cyclodextrin, β-cyclodextrin, salts, e.g. sodium chloride, chelators, e.g. EDTA as hereafter defined.

The term "sugar" as used herein denotes an oligosaccharide. An oligosaccharide is a carbohydrate consisting of more than one monomeric saccharide unit connected via glycosidic bond(s) either branched or in a chain. The monomeric saccharide units within an oligosaccharide can be identical or different. Depending on the number of monomeric saccharide units the oligosaccharide is a di-, tri-, tetra- penta- and so forth saccharide. In contrast to polysaccharides oligosaccharides are water soluble. Examples of oligosaccharides include sucrose, trehalose and raffinose.

The term "amino acid" as used herein denotes a pharmaceutically acceptable organic molecule possessing an amino moiety located at α-position to a carboxylic group. Examples of amino acids include arginine, glycine, histidine, tryptophane and methionine.

The term "polyols" as used herein denotes pharmaceutically acceptable alcohols with more than one hydroxy group. Suitable polyols comprise but are not limited to mannitol, sorbitol, and combinations thereof.

A subgroup within the stabilizers are antioxidants. The term "antioxidant" denotes pharmaceutically acceptable excipients, which prevent oxidation of the active pharmaceutical ingredient. Antioxidants comprise but are not limited to ascorbic acid, glutathione, cysteine, methionine, citric acid, EDTA.

The term "tonicity agents" as used herein denotes pharmaceutically acceptable tonicity agents. Tonicity agents are used to modulate the tonicity of the formulation. The formulation can be hypotonic, isotonic or hypertonic. Isotonicity in general relates to the osmotic pressure relative of a solution usually relative to that of human blood serum. The formulation according to the invention may be hypotonic, isotonic or hypertonic, e.g. be isotonic. Suitable tonicity agents comprise but are not limited to sodium chloride, and any component from the group of amino acids and sugars.

Within the stabilizers and tonicity agents there is a group of compounds which can function in both ways, i.e. they can at the same time be a stabilizer and a tonicity agent. Examples thereof can be found in the group of sugars, amino acids, polyols, cyclodextrines and salts, e.g. sodium chloride. An example for a sugar which can at the same time be a stabilizer and a tonicity agent is trehalose.

The compositions may also contain adjuvants such as preservatives, wetting agents, emulsifying agents and dispersing agents. Prevention of presence of microorganisms may be ensured both by sterilization procedures, and by the inclusion of various antibacterial and antifungal agents, for example, paraben, chlorobutanol, phenol, sorbic acid, and the like. Preservatives comprise but are not limited to ethanol, benzyl alcohol, phenol, m-cresol, p-chlor-m-cresol, methyl or propyl parabens, benzalkonium chloride.

The abovementioned buffers are generally used in an amount of about 1mM to about 100 mM, for example in an amount of about 5 mM to about 50 mM or in an amount of about 10 mM to 20 mM.

When polysorbate 20 (Tween 20™) and polysorbate 80 (Tween 80™) are used as surfactants they are generally used in a concentration range of about 0.005 to about 0.2% or of about 0.01% to about 0.1%w/v (weight / volume).

The stabilizers may be present in the formulation in an amount of about 10 to about 500 mM, e.g. in an amount of about 10 to about 300 mM or in an amount of about 100 mM to about 300 mM.

Amino acids are generally used in an amount of about 10 to 500 mM, e.g. in an amount of about 10 to about 300 mM or in an amount of about 100 to about 300 mM.

Polyols can be used in an amount of about 10 mM to about 500 mM, e.g. in an amount of about 10 to about 300 mM or in an amount of about 100 to about 300 mM.

Antioxidants can be used in an amount of about 1 to about 100 mM, e.g., in an amount of about 5 to about 50 mM or in an amount of about 5 to about 20 mM.

Preservatives may generally be used in an amount of about 0.001 to about 2 %(w/v).

The invention relates to a pharmaceutical liquid formulation of a human antibody binding to human OX40L comprising a buffer selected from histidine acetate and sodium acetate. The invention relates to a pharmaceutical liquid formulation of a human antibody binding to human OX40L comprising histidine acetate in an amount of 20 mM, at pH 5.8.

The invention relates to a pharmaceutical liquid formulation of a human antibody binding to human OX40L comprising a surfactant selected from polysorbate 20 (sold under the trademark Tween 20™), polysorbate 80 (sold under the trademark Tween 80™) and a polyethylene-polypropylene copolymer sold under the name Poloxamer 188™. The invention relates to a pharmaceutical liquid formulation of a human antibody binding to human OX40L comprising a surfactant selected from polysorbate 20 (sold under the trademark Tween 20™) and a polyethylene-polypropylene copolymer sold under the name Poloxamer 188™. The invention relates to a pharmaceutical liquid formulation of a human antibody binding to human OX40L comprising polysorbate 20 (sold under the trademark Tween 20™) in a concentration range of about 0.005 to about 0.2% or of about 0.01% to about 0.1%w/v (weight / volume).

The invention relates to a pharmaceutical liquid formulation of a human antibody binding to human OX40L comprising a stabilizer selected from the group consisting of sugars and amino acids, e.g. selected from sucrose, trehalose and raffmose, e.g. from sucrose and trehalose, e.g. trehalose in an amount of about 200 mM.

The invention relates to a pharmaceutical liquid formulation of a human antibody binding to human OX40L comprising a stabilizer and a tonicity agent wherein the stabilizer is selected from the group consisting of sugars and amino acids; and the tonicity agent is selected from the group consisting of sodium chloride, amino acids and sugars.

The invention relates to a pharmaceutical liquid formulation of a human antibody binding to human OX40L comprising a stabilizer and a tonicity agent wherein the stabilizer is a sugar, e.g. selected from sucrose, trehalose and raffinose, e.g. from sucrose and trehalose, e.g. trehalose; in an amount of about 200 mM, or an amino acid selected from arginine, glycine, histidine, tryptophane and methionine, e.g. arginine and methionine, e.g. methionine; e.g. in an amount of about 10 to 500 mM, e.g. in an amount of about 10 to about 300 mM, e.g. methionine in an amount of 10 mM, or in an amount of about 100 to about 300 mM, e.g. arginine in an amount of 130 mM; and the tonicity agent is selected from the group consisting of sodium chloride, amino acids and sugars, e.g. from trehalose and arginine.

The invention relates to a pharmaceutical liquid formulation of a human antibody binding to human OX40L comprising a stabilizer and a tonicity agent wherein the stabilizer is methionine in an amount of 10 mM; and the tonicity agent is selected from trehalose and arginine, e.g. in an amount of from about 100 to about 300 mM, e.g. trehalose in an amount of about 200 mM or arginine in an amount of about 130 mM.

The invention relates to a pharmaceutical liquid formulation of a human antibody binding to human OX40L comprising 20 to 200 mg/mL, e.g., from 20 to 160 mg/mL of an OX40L human antibody comprising as light chain the light chain of SEQ ID NO:1 and as heavy chain the heavy chain of SEQ ID NO:2 or 3.

The invention relates to a pharmaceutical liquid formulation of a human antibody binding to human OX40L comprising

1 to 200 mg/ml of said antibody comprising as light chain the light chain of SEQ ID NO:1 and as heavy chain the heavy chain of SEQ ID NO:2 or 3;
20 mM of a buffer selected from histidine acetate or sodium acetate, e.g. histidine acetate in an amount of 20 mM;
0.001 to 1% of a surfactant;

(a) 5 to 500 mM of a stabilizer; or
(b) 5 to 500 mM of a stabilizer and 5 to 500 mM of a tonicity agent; or
(c) 5 to 500 mM of a tonicity agent;
at pH 5.8.

The invention relates to a pharmaceutical liquid formulation of a human antibody binding to human OX40L comprising

1 to 200 mg/ml of said antibody comprising as light chain the light chain of SEQ ID NO:1 and as heavy chain the heavy chain of SEQ ID NO:2 or 3;
1 to 100 mM of a buffer;
0.001 to 1% of a surfactant;
5 to 500 mM of a stabilizer and 5 to 500 mM of a tonicity agent, wherein the stabilizer is selected from the group consisting of sugars and amino acids; and the tonicity agent is selected from the group consisting of sodium chloride, amino acids and sugars, e.g. a sugar as stabilizer, e.g. selected from sucrose, trehalose and raffinose, e.g. from sucrose and trehalose, e.g. trehalose, in an amount of about 200 mM, or an amino acid as stabilizer selected from arginine, glycine, histidine, tryptophane and methionine, e.g. arginine and methionine, e.g. methionine, e.g. in an amount of about 10 to 500 mM, e.g. in an amount of about 10 to about 300 mM, e.g. methionine in an amount of 10 mM, or in an amount of about 100 to about 300 mM, e.g. arginine in an amount of about 130 mM; and the tonicity agent is selected from the group consisting of sodium chloride, amino acids and sugars;
at a pH in the range of from 4.0 to 7.0.

The invention relates to a pharmaceutical liquid formulation of a human antibody binding to human OX40L comprising

1 to 200 mg/ml of said antibody comprising as light chain the light chain of SEQ ID NO:1 and as heavy chain the heavy chain of SEQ ID NO:2 or 3;
1 to 100 mM of a buffer selected from sodium acetate-buffer at pH 5.3 and histidine acetate buffer at pH 5.8;
0.001 to 1 % of a surfactant;

(a) 5 to 500 mM of a stabilizer selected from methionine, trehalose and arginine HCl; or
(b) 5 to 500 mM of methionine and 5 to 500 mM of a tonicity agent selected from trehalose and arginine HCl; or
(c) 5 to 500 mM of a tonicity agent selected from trehalose and arginine HCl.

The invention relates to a pharmaceutical liquid formulation of a human antibody binding to human OX40L comprising

1 to 200 mg/ml of said antibody comprising as light chain the light chain of SEQ ID NO:1 and as heavy chain the heavy chain of SEQ ID NO:2 or 3;
20 mM of a buffer selected from sodium acetate-buffer at pH 5.3 and histidine acetate buffer at pH 5.8;
0.001 to 1 % of a surfactant;

(a) a stabilizer selected from 10 mM methionine, 200 mM trehalose and 130 mM arginine HCl; or
(b) 10 mM methionine and a tonicity agent selected from 200 mM trehalose and 130 mM arginine HCl; or
(c) a tonicity agent selected from 200 mM trehalose and 130 mM arginine HCl.

The invention relates to a pharmaceutical liquid formulation of a human antibody binding to human OX40L comprising
1 to 200 mg/ml of said antibody comprising as light chain the light chain of SEQ ID NO:1 and as heavy chain the heavy chain of SEQ ID NO:2 or 3;
20 mM of a buffer selected from sodium acetate-buffer at pH 5.3 and histidine acetate buffer at pH 5.8;
0.05 % of a surfactant selected from Polysorbate 20, Polysorbate 80 and Poloxamer 188;

(a) a stabilizer selected from methionine, trehalose and arginine HCl; or
(b) methionine and a tonicity agent selected from trehalose and arginine HCl; or
(c) a tonicity agent selected from trehalose and arginine HCl.

The invention relates to a pharmaceutical liquid formulation of a human antibody binding to human OX40L comprising 150 mg/ml of said antibody comprising as light chain the light chain of SEQ ID NO:1 and as heavy chain the heavy chain of SEQ ID NO:2 or 3;

(i) 20 mM sodium acetate-buffer at pH 5.3;
0.05 % of Polysorbate 20;
10 mM methionine;
200 mM trehalose; or
(ii) 20 mM histidine acetate buffer at pH 5.8;
0.05 % of Polysorbate 20; and
200 mM trehalose; or
(iii) 20 mM of histidine acetate buffer at pH 5.8;
0.05 % of Polysorbate 80; and
200 mM trehalose; or
(iv) 20 mM of histidine acetate buffer at pH 5.8;
0.05 % of Polysorbate 20; and
130 mM arginine HCl; or
(v) 20 mM of histidine acetate buffer at pH 5.8;
0.05 % of Polysorbate 20;
10 mM methionine; and
200 mM trehalose; or
(vi) 20 mM of histidine acetate buffer at pH 5.8;
0.05 % of Poloxamer 188;
10 mM methionine; and
200 mM trehalose.

The human monoclonal antibodies against OX40L may be produced by recombinant means, e.g. by those described in WO2006/029879. Such methods are widely known in the art and comprise protein expression in prokaryotic and eukaryotic cells with subsequent isolation of the antibody polypeptide and usually purification to a pharmaceutically acceptable purity. For the protein expression, nucleic acids encoding light and heavy chains or fragments thereof are inserted into expression vectors by standard methods. Expression is performed in appropriate host cells like CHO cells, NS0 cells, SP2/0 cells, HEK293 cells, COS cells, and the antibody is recovered from the cells (supernatant or cells after lysis) by standard techniques, like column chromatography and others well known in the art, e.g. as described in WO2006/029879.

The formulation according to the invention can be prepared by methods known in the art, e.g. ultrafiltration-diafiltration, dialysis, addition and mixing, and combinations thereof. Examples of preparations of formulations according to the invention can be found hereinafter.

A composition of the present invention may be administered by a variety of methods known in the art. As will be appreciated by the skilled artisan, the route and/or mode of administration will vary depending upon the desired results.

To administer a composition of the invention by certain routes of administration, it may be necessary to dilute the composition in a diluent. Pharmaceutically acceptable diluents include saline, glucose, Ringer and aqueous buffer solutions.

The formulation according to the invention may be administered by intravenous (i.v.), subcutaneous (s.c.) or any other parental administration means such as those known in the pharmaceutical art.

The composition must be sterile and fluid to the extent that the composition is deliverable by parenteral administration, e.g., via a syringe needle or a cannula.

The formulations according to the invention are useful for prevention and/or treatment of inflammatory diseases in a mammal, e.g. a patient suspected of having or suffering from such a disease. Examples of such diseases include allergic reactions, e.g., asthma or allergic rhinitis.

For example, the formulations of the present invention may be used for the treatment of allergic rhinitis or asthma, e.g. moderate asthma, moderate to severe asthma or persistent asthma in patients whose symptoms are not adequately controlled with inhaled corticosteroids. The patient population may include adults and adolescents (12 years of age and older). The formulations may be delivered, e.g., subcutaneously, e.g. once or twice a month.

For example, the formulation of the present invention may be supplied in 2 ml single use vials as e.g. 1.25 ml of a 150 mg/ml liquid for injection (1 ml extractable volume). An exemplary administration procedure may be as follows: The vial is allowed to warm up to room temperature. A transfer needle is attached to a disposable 1 ml syringe. The whole content of the vial is withdrawn into the syringe. The transfer needle is removed and an injection needle for subcutaneous administration is attached. The formulation of the present invention is administered subcutaneously, in the right or left arm/thigh, at room temperature. Main endpoint may, e.g., be decrease in acute exacerbations. Other endpoints include peak flow, daytime asthma symptoms, nocturnal awakenings, quality of life, emergency room visits, asthma free days, beta-2 agonist use, steroid reduction or tapering and effect on hyper-responsiveness.

Examples

The following Examples are examples of the formulations of the invention. It is understood that various other embodiments may be practiced, given the general description provided above.

**Example 1: Preparation of liquid formulations**

Mab1 was provided at a concentration of approx 20 - 40mg/mL Mab1 in a 20mM histidine buffer at a pH of approx. 5.5 - 6.0. Liquid formulations of Mab1 were prepared by diafiltration against a diafiltration buffer containing the anticipated buffer composition and where required by ultrafiltration to increase the Mab1 protein concentration to approx. 200 mg/mL. Excipients for stabilizing the antibody and for tonicity adjustment as well as surfactants were added as stock solutions to the antibody solution as required. Finally the Mab1 protein concentration was adjusted to the desired concentration by dilution with buffer to the final Mab concentration of approx. 20 mg/mL or 150 mg/mL.

All formulations were sterile-filtered through 0.22 μm low protein binding filters and aseptically filled into sterile 6 mL glass vials closed with ETFE (Copolymer of ethylene and tetrafluoroethylene)-coated rubber stoppers and alucrimp caps. The fill volume was approx. 2.4 mL.

Liquid formulations of 150 mg Mab/ml

|   | Buffer [20 mM] | pH | surfactant [%] | methionine [mM] | Excipient [mM] |
|---|---|---|---|---|---|
| 1 | sodium acetate | 5.3 | 0.05 PS20 | 0 | 200 trehalose |
| 2 | sodium acetate | 5.3 | 0.05 PX188 | 0 | 200 trehalose |
| 3 | sodium acetate | 5.3 | 0.05 PS80 | 0 | 200 trehalose |
| 4 | sodium acetate | 5.3 | 0.03 PX188 | 0 | 200 trehalose |
| 5 | sodium acetate | 5.3 | 0.05 PS20 | 0 | 130 arginine HCl |
| 6 | sodium acetate | 5.3 | 0.05 PX188 | 0 | 130 arginine HCl |
| 7 | sodium acetate | 5.3 | 0.05 PX188 | 10 | 200 trehalose |

(continued)

| | Buffer [20 mM] | pH | surfactant [%] | methionine [mM] | Excipient [mM] |
|---|---|---|---|---|---|
| 8 | sodium acetate | 5.3 | 0.05 PS20 | 10 | 200 trehalose |
| 9 | histidine acetate | 5.8 | 0.05 PS20 | 0 | 200 trehalose |
| 10 | histidine acetate | 5.8 | 0.05 PX188 | 0 | 200 trehalose |
| 11 | histidine acetate | 5.8 | 0.05 PS80 | 0 | 200 trehalose |
| 12 | histidine acetate | 5.8 | 0.03 PX188 | 0 | 200 trehalose |
| 13 | histidine acetate | 5.8 | 0.05 PS20 | 0 | 130 arginine HCl |
| 14 | histidine acetate | 5.8 | 0.05 PX188 | 0 | 130 arginine HCl |
| 15 | histidine acetate | 5.8 | 0.05 PS20 | 10 | 200 trehalose |
| 16 | histidine acetate | 5.8 | 0.05 PX188 | 10 | 200 trehalose |
| PS20: Polysorbate 20, PX188: Poloxamer 188 | | | | | |

## Example 2: Stability of liquid formulations

The formulations according to Example 1 were stored at different ICH climate conditions (2 to 8°C and 40°C) for different intervals of time and analyzed by 1) UV spectrophotometry, and 2) Size Exclusion Chromatography (SEC).

Size Exclusion Chromatography (SEC) was used to detect soluble high molecular weight species (HMW aggregates) and low molecular weight hydrolysis products (LMW) in the formulations. Analysis was performed on a Water Alliance 2795 HPLC instrument equipped with a TSKgel G3000 SWXL column (7.8x300mm). Intact monomer, aggregates and hydrolysis products were separated by an isocratic elution profile using 0.2M $K_2HPO_4$ / 0.25M KCL, pH 7.0 as mobile phase, and were detected at a wavelength of 280nm. UV spectroscopy, used for determination of protein content, was performed on a Varian Cary Bio UV spectrophotometer in a wavelength range from 240 nm to 400 nm. Neat protein samples were diluted to approx. 0.5 mg/mL with the corresponding formulation buffer. The protein concentration was calculated according to equation 1.

$$\text{Equation 1:} \qquad \text{Protein content} = \frac{A(280) - A(320) \times dil.factor}{\varepsilon \left\langle \frac{cm^2}{mg} \right\rangle \times d \langle cm \rangle}$$

The UV light absorption at 280 nm was corrected for light scattering at 320 nm and multiplied with the dilution factor, which was determined from the weighed masses and densities of the neat sample and the dilution buffer. The numerator was divided by the product of the cuvette's path length d and the extinction coefficient $\varepsilon$.

Table 1:

| Formulation 15 | | | | |
|---|---|---|---|---|
| Storage at 2-8°C | | | | |
| Timepoint | Protein conc. (mg/mL) | Size Exclusion - HPLC | | |
| | | HMW (%) | Monomer (%) | LMW (%) |
| Initial | 156.4 | 0.9 | 99.1 | 0 |
| 1 months | 155.4 | 1.1 | 98.9 | 0 |
| 3 months | 156.6 | 1.1 | 98.8 | 0.1 |
| 6 months | 156.4 | 1.2 | 98.7 | 0.1 |

(continued)

| Storage at 40°C | | | | |
|---|---|---|---|---|
| Timepoint | Protein conc. (mg/mL) | Size Exclusion - HPLC HMW (%) | Monomer (%) | LMW (%) |
| Initial | 156.4 | 0.9 | 99.1 | 0 |
| 1 months | n/a | 2.2 | 94.7 | 3.2 |
| 3 months | n/a | 2.4 | 93.1 | 4.5 |
| 6 months | n/a | 3.3 | 89.0 | 7.7 |

**Formulation 9**

| Storage at 2-8°C | | | | |
|---|---|---|---|---|
| Timepoint | Protein conc. (mg/mL) | Size Exclusion - HPLC HMW (%) | Monomer (%) | LMW (%) |
| Initial | 156.1 | 1.0 | 99.0 | 0 |
| 1 months | 158.2 | 1.2 | 98.7 | 0.1 |
| 3 months | 155.3 | 1.3 | 98.6 | 0.1 |
| 6 months | 153.4 | 1.5 | 98.4 | 0.1 |

| Storage at 40°C | | | | |
|---|---|---|---|---|
| Timepoint | Protein conc. (mg/mL) | Size Exclusion - HPLC HMW (%) | Monomer (%) | LMW (%) |
| Initial | 156.1 | 1.0 | 99.0 | 0 |
| 1 months | n/a | 2.9 | 93.9 | 3.2 |
| 3 months | n/a | 3.4 | 92.2 | 4.4 |
| 6 months | n/a | 4.9 | 87.6 | 7.5 |

**Formulation 13**

| Storage at 2-8°C | | | | |
|---|---|---|---|---|
| Timepoint | Protein conc. (mg/mL) | Size Exclusion - HPLC HMW (%) | Monomer (%) | LMW (%) |
| Initial | 158.9 | 0.9 | 99.1 | 0 |
| 1 months | 155.4 | 0.9 | 99.1 | 0 |
| 3 months | 160.7 | 1.0 | 99.0 | 0 |
| 6 months | 157.8 | 1.0 | 98.1 | 0.1 |

| Storage at 40°C | | | | |
|---|---|---|---|---|
| Timepoint | Protein conc. (mg/mL) | Size Exclusion - HPLC HMW (%) | Monomer (%) | LMW (%) |
| Initial | 158.9 | 0.9 | 99.1 | 0 |
| 1 months | n/a | 2.0 | 96.2 | 1.8 |
| 3 months | n/a | 3.4 | 91.4 | 5.2 |

(continued)

| Storage at 40°C | | | | |
|---|---|---|---|---|
| Timepoint | Protein conc. (mg/mL) | Size Exclusion - HPLC | | |
| | | HMW (%) | Monomer (%) | LMW (%) |
| 6 months | n/a | 6.1 | 84.4 | 9.6 |
| **Formulation 8** | | | | |
| Storage at 2-8°C | | | | |
| Timepoint | Protein conc. (mg/mL) | Size Exclusion - HPLC | | |
| | | HMW (%) | Monomer (%) | LMW (%) |
| Initial | 159.1 | 0.7 | 99.3 | 0 |
| 2 months | 160.5 | 1.1 | 98.9 | 0 |
| 3 months | 162.2 | 1.1 | 98.9 | 0 |
| Storage at 40°C | | | | |
| Timepoint | Protein conc. (mg/mL) | Size Exclusion - HPLC | | |
| | | HMW (%) | Monomer (%) | LMW (%) |
| Initial | 159.1 | 0.7 | 99.3 | 0 |
| 2 months | n/a | 2.3 | 94.5 | 3.2 |
| 3 months | n/a | 2.6 | 92.8 | 4.6 |
| **Formulation 11** | | | | |
| Storage at 2-8°C | | | | |
| Timepoint | Protein conc. (mg/mL) | Size Exclusion - HPLC | | |
| | | HMW (%) | Monomer (%) | LMW (%) |
| Initial | 156.5 | 1.0 | 99.0 | 0 |
| 2 months | 156.5 | 1.3 | 98.7 | 0 |
| 3 months | 154.6 | 1.3 | 98.6 | 0.1 |
| Storage at 40°C | | | | |
| Timepoint | Protein conc. (mg/mL) | Size Exclusion - HPLC | | |
| | | HMW (%) | Monomer (%) | LMW (%) |
| Initial | 156.5 | 1.0 | 99.0 | 0 |
| 2 months | n/a | 3.2 | 93.4 | 3.4 |
| 3 months | n/a | 3.6 | 91.7 | 4.7 |
| **Formulation 16** | | | | |
| Storage at 2-8°C | | | | |
| Timepoint | Protein conc. (mg/mL) | Size Exclusion - HPLC | | |
| | | HMW (%) | Monomer (%) | LMW (%) |
| Initial | 155.5 | 0.9 | 99.1 | 0 |

(continued)

| Formulation 16 | | | | |
|---|---|---|---|---|
| Storage at 2-8°C | | | | |
| Timepoint | Protein conc. (mg/mL) | Size Exclusion - HPLC | | |
| | | HMW (%) | Monomer (%) | LMW (%) |
| 2 months | 157.2 | 1.1 | 98.9 | 0 |
| 3 months | 156.9 | 1.1 | 98.8 | 0.1 |
| Storage at 40°C | | | | |
| Timepoint | Protein conc. (mg/mL) | Size Exclusion - HPLC | | |
| | | HMW (%) | Monomer (%) | LMW (%) |
| Initial | 155.5 | 0.9 | 99.1 | 0 |
| 2 months | n/a | 2.1 | 94.7 | 3.2 |
| 3 months | n/a | 2.4 | 93.1 | 4.5 |

**Example 3: Comparison between liquid OX40L antibody formulations**

The following formulations were used:
Formulation A: 20 mg Liquid Formulation based on the lyophilized formulation disclosed in WO2009/141239, i.e. 20 mg/mL Mab1, 20 mM histidine/histidine HCl, 240 mM trehalose, 0.02% polysorbate 20, at pH 6.0
Formulation B: 150 mg Liquid Formulation based on the lyophilized formulation disclosed in WO2009/141239, i.e. 150 mg/mL Mab1, 20 mM histidine/histidine HCl, 240 mM trehalose, 0.02% polysorbate 20, at pH 6.0
Formulation C: Liquid Formulation of the present invention, i.e. 20 mg/mL Mab1, 20 mM histidine acetate, 200 mM trehalose, 10mM methionine, 0.05% polysorbate 20, at pH 5.8 Formulation D: Liquid Formulation of the present invention, i.e. 150 mg/mL Mab1, 20 mM histidine acetate, 200 mM trehalose, 10mM methionine, 0.05% polysorbate 20, at pH 5.8

| | Formulation A | | | | Formulation C | | | |
|---|---|---|---|---|---|---|---|---|
| | Storage at 2-8°C | | | | | | | |
| | Protein* | Size Exclusion - HPLC | | | Protein* | Size Exclusion - HPLC | | |
| Timepoint | | HMW | Mono | LMW | | HMW | Mono | LMW |
| Initial | 19.7 | 0.5 | 99.4 | 0.1 | 21.7 | 0.5 | 99.4 | 0.1 |
| 7 months | n/a | 0.6 | 99.3 | 0.1 | n/a | 0.5 | 99.4 | 0.1 |
| | Storage at 40°C | | | | | | | |
| | Protein* | Size Exclusion - HPLC | | | Protein* | Size Exclusion - HPLC | | |
| Timepoint | | HMW | Mono | LMW | | HMW | Mono | LMW |
| Initial | 19.7 | 0.5 | 99.4 | 0.1 | 21.7 | 0.5 | 99.4 | 0.1 |
| 7 months | n/a | 1.9 | 87.7 | 10.4 | n/a | 0.8 | 88.8 | 10.4 |
| | Formulation B | | | | Formulation D | | | |
| | Storage at 2-8°C | | | | | | | |
| | Protein* | Size Exclusion - HPLC | | | Protein* | Size Exclusion - HPLC | | |
| Timepoint | | HMW | Mono | LMW | | HMW | Mono | LMW |
| Initial | 159.9 | 1.0 | 98.9 | 0.1 | 160.5 | 1.0 | 98.9 | 0.1 |
| 7 months | n/a | 1.7 | 98.2 | 0.1 | n/a | 1.3 | 98.6 | 0.1 |

(continued)

| | Storage at 40°C | | | | | | | |
| | Protein* | Size Exclusion - HPLC | | | Protein* | Size Exclusion - HPLC | | |
| Timepoint | | HMW | Mono | LMW | | HMW | Mono | LMW |
| Initial | 159.9 | 1.0 | 98.9 | 0.1 | 160.5 | 1.0 | 98.9 | 0.1 |
| 7 months | n/a | 6.5 | 84.2 | 9.3 | n/a | 4.4 | 86.1 | 9.5 |
| *Protein (mg/mL); ** HMW: high molecular weight (%); Mono: Monomer (%); LMW: low molecular weight (%) | | | | | | | | |

**Claims**

1. A pharmaceutical liquid formulation comprising:

   1 to 200 mg/mL of an antibody;
   1 to 100 mM of a buffer;
   0.001 to 1% of a surfactant;

   (a) 5 to 500 mM of a stabilizer; or
   (b) 5 to 500 mM of a stabilizer and 5 to 500 mM of a tonicity agent; or
   (c) 5 to 500 mM of a tonicity agent;

   at a pH in the range of from 4.0 to 7.0,
   wherein the antibody is an antibody against OX40 ligand.

2. The formulation according to claim 1 wherein the antibody against OX40 ligand is a human antibody binding to human OX40L comprising as light chain the light chain of SEQ ID NO:1 and as heavy chain the heavy chain of SEQ ID NO:2 or 3.

3. The formulation according to any of claims 1 and 2 comprising a buffer selected from histidine acetate and sodium acetate.

4. The formulation according to claim 3 wherein the buffer is histidine acetate in an amount of 20 mM, at pH 5.8.

5. The formulation according to any of claims 3 and 4 comprising a surfactant selected from polysorbate 20 (sold under the trademark Tween 20™), polysorbate 80 (sold under the trademark Tween 80™) and a polyethylene-polypropylene copolymer sold under the name Poloxamer 188™.

6. The formulation according to any one of claims 1 to 5 comprising trehalose in an amount of about 200 mM as a stabilizer.

7. The formulation according to any one of claims 1 to 5 comprising arginine in an amount of about 130 mM as a stabilizer.

8. The formulation according to any one of claims 1 to 5 comprising a stabilizer and a tonicity agent wherein the stabilizer is selected from the group consisting of sugars and amino acids, and the tonicity agent is selected from the group consisting of sodium chloride, amino acids and sugars.

9. The formulation according to claim 8 wherein the stabilizer is present in an amount of about 10 to about 500 mM.

10. The formulation according to any one of claims 8 and 9 wherein the stabilizer is selected from sucrose, trehalose, raffinose, arginine, glycine, histidine, tryptophane and methionine, and the tonicity agent is selected from trehalose and arginine.

11. The formulation according to claim 10 wherein the stabilizer is methionine in an amount of 10 mM; and the tonicity agent is selected from trehalose in an amount of about 200 mM and arginine in an amount of about 130 mM.

12. The formulation according to any one of claims 1 and 5 comprising a tonicity agent selected from the group consisting of amino acids and sugars.

13. The formulation according to claim 12 wherein the tonicity agent is trehalose in an amount of 200 mM.

14. The formulation according to claim 12 wherein the tonicity agent is arginine in an amount of 130 mM.

15. The formulation according to any one of claims 1 to 14 comprising 20 mM of a buffer selected from histidine acetate and sodium acetate.

16. The formulation according to claim 15, comprising histidine acetate in an amount of 20 mM, at pH 5.8.

17. The formulation according to claim 15, comprising sodium acetate in an amount of 20 mM, at pH 5.3.

18. The formulation according to any one of claims 1 or 2 comprising
1 to 100 mM of a buffer selected from sodium acetate-buffer at pH 5.3 and histidine acetate buffer at pH 5.8;

(a) 5 to 500 mM of a stabilizer selected from methionine, trehalose and arginine HCl; or
(b) 5 to 500 mM of methionine and 5 to 500 mM of a tonicity agent selected from trehalose and arginine HCl; or
(c) 5 to 500 mM of a tonicity agent selected from trehalose and arginine HCl.

19. The formulation according to claim 18 comprising
20 mM of a buffer selected from sodium acetate-buffer at pH 5.3 and histidine acetate buffer at pH 5.8;
0.001 to 1 % of a surfactant;

(a) a stabilizer selected from 10 mM methionine, 200 mM trehalose and 130 mM arginine HCl; or
(b) 10 mM methionine and a tonicity agent selected from 200 mM trehalose and 130 mM arginine HCl; or
(c) a tonicity agent selected from 200 mM trehalose and 130 mM arginine HCl.

20. The formulation according to any one of claims 1 to 19 comprising 150 mg/ml of the human antibody binding to human OX40L comprising as light chain the light chain of SEQ ID NO:1 and as heavy chain the heavy chain of SEQ ID NO:2 or 3;

(i) 20 mM sodium acetate-buffer at pH 5.3;
0.05 % of Polysorbate 20;
10 mM methionine; and
200 mM trehalose; or
(ii) 20 mM histidine acetate buffer at pH 5.8;
0.05 % of Polysorbate 20; and
200 mM trehalose; or
(iii) 20 mM of histidine acetate buffer at pH 5.8;
0.05 % of Polysorbate 80; and
200 mM trehalose; or
(iv) 20 mM of histidine acetate buffer at pH 5.8;
0.05 % of Polysorbate 20; and
130 mM arginine HCl; or
(v) 20 mM of histidine acetate buffer at pH 5.8;
0.05 % of Polysorbate 20;
10 mM methionine; and
200 mM trehalose; or
(vi) 20 mM of histidine acetate buffer at pH 5.8;

0.05 % of Poloxamer 188;
10 mM methionine; and
200 mM trehalose.

21. The formulation according to any one of claims 1 to 20 for use in the therapeutic treatment of an inflammatory disease in a mammal.

22. A method for the therapeutic and/or prophylactic treatment of an inflammatory disease, particularly for the treatment of asthma, which method comprises administering a liquid formulation according to any one of claims 1 to 20 to a human being or animal.

Europäisches
Patentamt

European
Patent Office

Office européen
des brevets

# EUROPEAN SEARCH REPORT

Application Number

EP 10 16 7357

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/141239 A1 (HOFFMANN LA ROCHE [CH]; ADLER MICHAEL [DE]; MAHLER HANNS-CHRISTIAN [CH] 26 November 2009 (2009-11-26) * page 4, line 10 - line 13; claims; examples; sequences 58,61 * | 1,2,6, 8-10,12, 13,21,22 | INV. A61K39/395 C07K16/28 A61P11/06 |
| Y | * page 7, line 14 - page 10, line 5 * | 3-5,7, 11, 14-16, 18-20 | |
| Y | WO 2006/044908 A2 (GENENTECH INC [US]; ANDYA JAMES D [US]; GWEE SHIANG C [US]; LIU JUN [U) 27 April 2006 (2006-04-27) <br><br> * page 8, paragraph 1 - paragraph 5; claims; examples * <br> * page 12, paragraph 8 - page 13, paragraph 1 * <br> * page 33, paragraph 2 * <br> * page 55, paragraph 4 - page 56, paragraph 4 * <br> * page 57, paragraph 6 - page 58, paragraph 6 * <br> * page 59, paragraph 6 * <br> * page 62, paragraph 5 * | 3-5,7, 11, 14-16, 18-20 | |
| A | WO 2006/029879 A2 (HOFFMANN LA ROCHE [CH]; ENDL JOSEF [DE]; EUGUI ELSIE [US]; FUENTES MAR) 23 March 2006 (2006-03-23) * page 6, line 4 - line 7 * * page 37, line 6 - line 20 * | 1-16, 18-22 | TECHNICAL FIELDS SEARCHED (IPC) <br><br> A61K <br> C07K |
| A | WO 2010/059787 A1 (GENENTECH INC [US]; KABAKOFF BRUCE [US]; NELSON DAREN [US]; OUYANG JUN) 27 May 2010 (2010-05-27) * claims; examples * | 1-16, 18-22 | |

-/--

~~The present search report has been drawn up for all claims~~

| Place of search | Date of completion of the search | Examiner |
|---|---|---|
| Munich | 24 November 2010 | Loubradou, Gabriel |

EPO FORM 1503 03.82 (P04C01)

Europäisches
Patentamt

European
Patent Office

Office européen
des brevets

# EUROPEAN SEARCH REPORT

Application Number

EP 10 16 7357

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2004/197324 A1 (LIU JUN [US] ET AL) 7 October 2004 (2004-10-07) * claims; examples * * page 1, right-hand column, paragraph 0012 - paragraph 0013 * ----- | 1-16, 18-22 | |
| A | WO 2007/109221 A2 (WYETH CORP [US]; WARNE NICHOLAS WILLIAM [US]; KANTOR ANGELA [US]; CROW) 27 September 2007 (2007-09-27) * page 24, paragraph 3 - page 25, paragraph 1; claims; examples * ----- | 1-16, 18-22 | |

TECHNICAL FIELDS
SEARCHED (IPC)

~~The present search report has been drawn up for all claims~~

| Place of search | Date of completion of the search | Examiner |
|---|---|---|
| Munich | 24 November 2010 | Loubradou, Gabriel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................................

& : member of the same patent family, corresponding
    document

EPO FORM 1503 03.82 (P04C01)

Europäisches
Patentamt

European
Patent Office

Office européen
des brevets

**Application Number**

EP 10 16 7357

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-16, 18-22(all partially)

☐ The present supplementary European search report has been drawn up for those parts of the European patent application which relate to the invention first mentioned in the claims (Rule 164 (1) EPC).

22

Europäisches
Patentamt

European
Patent Office

Office européen
des brevets

**LACK OF UNITY OF INVENTION**
**SHEET B**

Application Number

EP 10 16 7357

The Search Division considers that the present European patent application does not comply with the
requirements of unity of invention and relates to several inventions or groups of inventions, namely:

1. claims: 1-16, 18-22(all partially)

   Subject-matter of claims 1 to 16 and 18 to 22 wherein the
   antibody comprises the light chain of SEQ ID N°1 and the
   heavy chain of SEQ ID N°2 and wherein the buffer is
   histidine acetate.
   ---

2. claims: 1-3, 5-15, 17-22(all partially)

   Subject-matter of claims 1 to 3, 5 to 15 and 17 to 22
   wherein the antibody comprises the light chain of SEQ ID N°1
   and the heavy chain of SEQ ID N°2 and wherein the buffer is
   sodium acetate.
   ---

3. claims: 1-22(partially)

   Subject-matter of claims 1 to 22 wherein the antibody
   comprises the light chain of SEQ ID N°1 and the heavy chain
   of SEQ ID N°3.
   ---

## ANNEX TO THE EUROPEAN SEARCH REPORT
## ON EUROPEAN PATENT APPLICATION NO.

EP 10 16 7357

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2010

| Patent document cited in search report | | Publication date | Patent family member(s) | | Publication date |
|---|---|---|---|---|---|
| WO 2009141239 | A1 | 26-11-2009 | AR | 071852 A1 | 21-07-2010 |
| | | | PE | 18522009 A1 | 31-12-2009 |
| | | | US | 2010098712 A1 | 22-04-2010 |
| WO 2006044908 | A2 | 27-04-2006 | AU | 2005295394 A1 | 27-04-2006 |
| | | | BR | PI0516299 A | 02-09-2008 |
| | | | CA | 2579861 A1 | 27-04-2006 |
| | | | CN | 101084015 A | 05-12-2007 |
| | | | CR | 9129 A | 25-05-2009 |
| | | | EC | SP077308 A | 26-04-2007 |
| | | | EP | 1802344 A2 | 04-07-2007 |
| | | | JP | 2008520551 T | 19-06-2008 |
| | | | KR | 20070068385 A | 29-06-2007 |
| | | | NZ | 553625 A | 30-10-2009 |
| | | | PA | 8650001 A1 | 17-01-2007 |
| | | | PE | 13272009 A1 | 03-09-2009 |
| | | | PE | 10432006 A1 | 19-10-2006 |
| | | | SV | 2006002275 A | 06-03-2006 |
| | | | ZA | 200702521 A | 30-07-2008 |
| WO 2006029879 | A2 | 23-03-2006 | AR | 051925 A1 | 21-02-2007 |
| | | | AU | 2005284310 A1 | 23-03-2006 |
| | | | BR | PI0515554 A | 29-07-2008 |
| | | | CA | 2580140 A1 | 23-03-2006 |
| | | | CN | 101684157 A | 31-03-2010 |
| | | | EP | 1791869 A2 | 06-06-2007 |
| | | | EP | 2218782 A2 | 18-08-2010 |
| | | | JP | 4594986 B2 | 08-12-2010 |
| | | | JP | 2008512995 T | 01-05-2008 |
| | | | JP | 2010280673 A | 16-12-2010 |
| | | | KR | 20070050972 A | 16-05-2007 |
| | | | KR | 20080059471 A | 27-06-2008 |
| | | | NZ | 553333 A | 25-09-2009 |
| | | | NZ | 579022 A | 30-04-2010 |
| | | | SG | 147444 A1 | 28-11-2008 |
| | | | US | 7501496 B1 | 10-03-2009 |
| | | | US | 2010166740 A1 | 01-07-2010 |
| WO 2010059787 | A1 | 27-05-2010 | NONE | | |
| US 2004197324 | A1 | 07-10-2004 | US | 2005158303 A1 | 21-07-2005 |
| | | | US | 2009280129 A1 | 12-11-2009 |
| | | | US | 2007053900 A1 | 08-03-2007 |
| | | | US | 2010158898 A1 | 24-06-2010 |
| WO 2007109221 | A2 | 27-09-2007 | AR | 059964 A1 | 14-05-2008 |

For more details about this annex : see Official Journal of the European Patent Office, No. 12/82

**ANNEX TO THE EUROPEAN SEARCH REPORT
ON EUROPEAN PATENT APPLICATION NO.**

EP 10 16 7357

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2010

| Patent document cited in search report | Publication date | Patent family member(s) | | Publication date |
|---|---|---|---|---|
| WO 2007109221 A2 | | AU | 2007227408 A1 | 27-09-2007 |
| | | CA | 2646934 A1 | 27-09-2007 |
| | | CN | 101420972 A | 29-04-2009 |
| | | EC | SP088758 A | 31-10-2008 |
| | | EP | 1996221 A2 | 03-12-2008 |
| | | JP | 2009530380 T | 27-08-2009 |
| | | KR | 20080108554 A | 15-12-2008 |
| | | PE | 01212008 A1 | 05-03-2008 |
| | | RU | 2008137634 A | 27-04-2010 |
| | | US | 2008064856 A1 | 13-03-2008 |

For more details about this annex : see Official Journal of the European Patent Office, No. 12/82

## REFERENCES CITED IN THE DESCRIPTION

**Patent documents cited in the description**

- WO 2006029879 A
- WO 2009141239 A

**Non-patent literature cited in the description**

- **CLELAND et al.** *Crit Rev Ther Drug Carrier Syst,* 1993, vol. 10 (4), 307-77
- **WANG.** *Int J Pharm,* 1999, vol. 185 (2), 129-88
- **WANG.** *Int J Pharm,* 2000, vol. 203 (1-2), 1-60
- **CHI et al.** *Pharm Res,* 2003, vol. 20 (9), 1325-36